# EUROPEAN PATENT SPECIFICATION

(11) **EP 1 574 184 B1**
(45) Date of publication and mention of the grant of the patent: **05.09.2007**
(21) Application number: 05002401.7
(22) Date of filing: 04.02.2005
(51) Int. Cl.: A61F 2/38

(54) **Tibial implant with a through post**
Tibialimplantat mit einem durchgeführten Stamm
Implant tibial avec une tige traversante

(30) Priority: 09.03.2004 US 797663
(43) Date of publication of application: 14.09.2005
(73) Proprietor: Zimmer Technology, Inc., Chicago, Illinois 60606 (US)
(72) Inventor: Daniels, Michael E., Warsaw Indiana 46580 (US)
(74) Representative: Manitz, Finsterwald & Partner GbR

(56) References cited:
- EP-A- 1 352 622
- US-A- 5 876 456
- US-A- 6 102 951

## Description

### FIELD OF THE INVENTION

The present invention relates generally to the field of orthopaedics, and, more particularly, to a tibial implant.

### BACKGROUND

Total joint arthroplasty ("joint replacement") is the surgical replacement of a joint with a prosthesis. A typical knee prosthesis has three main components: a femoral implant, a tibial implant, and a tibio-femoral insert. In general, the femoral implant is designed to replace the distal femoral condyles. The femoral implant is typically made from metal. It typically includes a head portion ("femoral head') having rounded surfaces for emulating the condyles, and an elongated stem extending away from the femoral head for anchoring the femoral implant in the intramedullary canal of the distal femur. In general, the tibial implant is designed to support and align the tibio-femoral insert. The tibial implant is also typically made from metal. It typically includes a substantially planar tray or plate portion ("tibial plate") for supporting the insert, and an elongated stem extending away from the tibial plate for anchoring the tibial implant in the intramedullary canal of the proximal tibia. In general, the tibio-femoral insert is designed to replace the tibial plateau and the meniscus of the knee. It is typically somewhat disk-shaped, and typically includes one or more substantially planar surfaces for bearing on the tibial plate and one or more generally concave surfaces for bearing against the femoral head. The insert is typically made of a strong, smooth, low-wearing plastic.

In a traditional knee replacement, the surgeon makes a rather lengthy anterior incision spanning over the distal femur, the knee, and the proximal tibia; separates the distal femur and proximal tibia from the surrounding tissues; hyperflexes, distally extends, and/or otherwise moves the proximal tibia away from the distal femur to make room for specialized guides and saws; and uses the guides and saws to prepare these bones for receiving the prosthetics. Finally, the surgeon drives the stems of the femoral implant and tibial implant generally longitudinally into the intramedullary canals of the distal femur and proximal tibia, respectively; cements each stem in place; aligns and/or attaches the insert onto the tibial plate; aligns and/or attaches the femoral head onto the insert; and closes the surgical site.

In contrast to a traditional knee replacement, knee replacement through minimally invasive surgery employs, among other things, smaller incisions, which tend to reduce tissue traumas and accelerate post-operative recoveries. However, because minimally invasive surgery reduces the size of the surgical site, it also generally reduces the amount of space available for inserting, aligning, and securing tibial implants having long, unitary stems.

US 6,102,951 discloses a tibial implant apparatus comprising a tibial plate coupled to the tibial plate. An elongated member is removably attached to a base extending from the tibial plate. The tibial plate and the base define a through channel, through which the elongated member is inserted such that a first portion is retained within the through channel and a second portion protrudes out of said through channel.

### SUMMARY OF THE INVENTION

It is the object of the present invention to provide an improved tibial implant apparatus. This object is achieved with the tibial implant apparatus having the features of claim 1. Subclaims are directed to preferable embodiments.

The inventive tibial implant apparatus is usable in connection with a method for anchoring a first member of a tibial implant and a second member of a tibial implant in the proximal tibia. This method includes the steps of anchoring the first member in the proximal tibia, anchoring the second member in the proximal tibia and coupling the second member to the first member simultaneously with the step of anchoring the second member and the proximal tibia. Preferably, the coupling step includes extending the second member through the first member.

The above-noted features and advantages of the present invention, as well as additional features and advantages will be readily apparent to those skilled in the art upon reference to the following detailed description and the accompanying drawings.

### BRIEF DESCRIPTION OF THE DRAWINGS

FIG. 1 shows a partially exploded perspective view of an exemplary apparatus according to the present invention;

FIG. 2 shows a superior plan view of the exemplary tibial implant of FIG. 1;

FIG. 3 shows an inferior plan view of the exemplary tibial plate and the exemplary base of FIG. 1;

FIG. 4 shows an exploded cross-sectional view of the exemplary tibial implant of FIG. 1 and FIG. 2 (taken along line 4-4 of FIG. 2); and

FIG. 5 shows an assembled cross-sectional view of the exemplary tibial implant of FIG. 1 and FIG. 2 (taken along line 4-4 of FIG. 2).

### DETAILED DESCRIPTION OF EXEMPLARY EMBODIMENT(S)

Like reference numerals refer to like parts throughout the following description and the accompanying drawings. As used herein, the terms "medial," "medially," and the like mean pertaining to the middle, in or toward the middle, and/or nearer to the middle of the body when standing upright. Conversely, the terms "lateral," "laterally," and the like are used herein as opposed to medial. For example, the medial side of the knee is the side closest to the other knee and the closest sides of the knees are medially facing, whereas the lateral side of the knee is the outside of the knee and is laterally facing. Further, as used herein the term "superior" means closer to the top of the head and/or farther from the bottom of the feet when standing upright. Conversely, the term "inferior" is used herein as opposed to superior. For example, the heart is superior to the stomach and the superior surface of the tongue rests against the palate, whereas the stomach is inferior to the heart and the palate faces inferiorly toward the tongue. Additionally, as used herein the terms "anterior," "anteriorly," and the like mean nearer the front or facing away from the front of the body when standing upright, as opposed to "posterior," "posteriorly," and the like, which mean nearer the back or facing away from the back of the body.

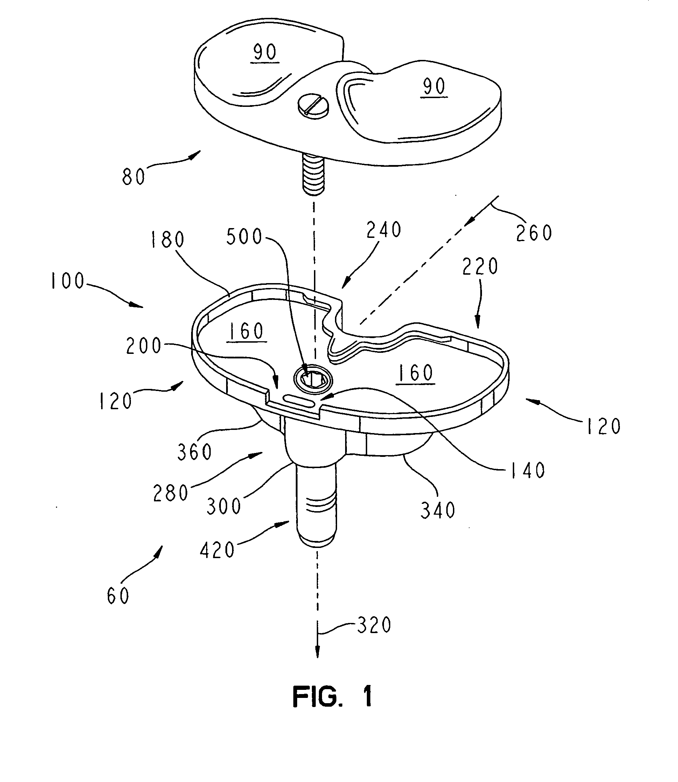

FIG. 1 shows a partially exploded perspective view of an exemplary apparatus 60 according to the present invention. Apparatus 60 includes an exemplary tibio-femoral insert 80. Insert 80 includes a pair of smooth surfaces 90 configured to serve as bearing surfaces against a femoral head (not shown). In the exemplary embodiment, insert 80 is made from Ultra High Density Polyethylene ("UHDPE"). In alternative embodiments, insert 80 may be made from any other suitably strong, smooth, low-wearing biocompatible material(s).

Apparatus 60 further includes an exemplary tibial implant 100. Among other things, implant 100 is configured to support and align insert 80. In the exemplary embodiment, implant 100 is made from a titanium alloy. In alternative embodiments, implant 100 may be made from a cobalt chrome alloy or any other suitable biocompatible material(s). Exemplary implant 100 includes an exemplary tibial plate 120. Among other things, plate 120 is configured to support insert 80. Plate 120 defines a trough or slot 140 and includes a generally planar surface 160 surrounding slot 140. Plate 120 further includes a retaining wall 180 substantially bounding and extending generally perpendicularly away from surface 160. Wall 180 defines a notch 200. It should be appreciated that plate 120 may be thought of as having a medial side or lobe 220 and an opposing lateral side or lobe 240 (relative to an imaginary split line 260). Additionally, it should be appreciated that slot 140, surface 160, wall 180 and notch 200 may facilitate alignment and/or retention of insert 80.

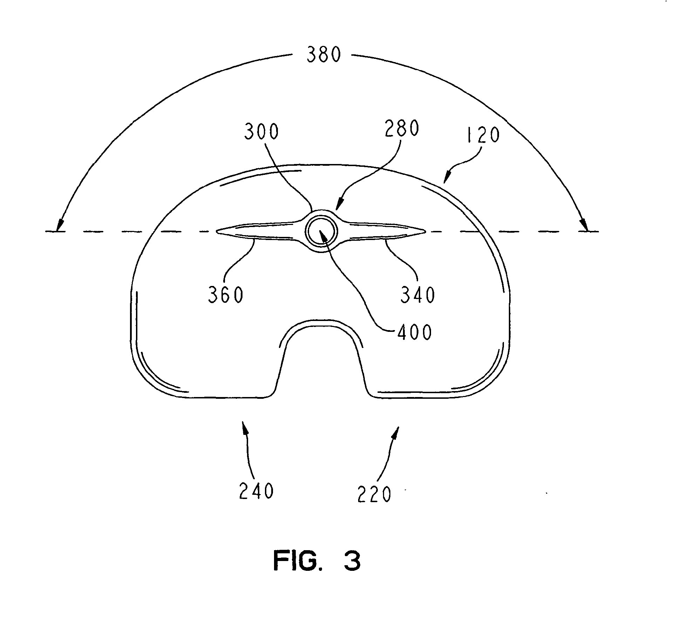

Implant 100 also includes an exemplary base 280 extending from plate 120. Among other things, base 280 is configured to anchor into a proximal tibia (not shown) such that base 280 (and, thus, plate 120) is rotationally fixed relative to the proximal tibia about axis 320. Base 280 is a generally keel-like structure extending generally inferiorly from and generally medially-laterally relative to plate 120. Further, base 280 includes a collar portion 300 extending generally inferiorly from plate 120 along an axis 320, further includes a medial generally keel-like portion 340 extending generally inferiorly from plate 120 and radiating generally medially from collar 300, and further includes a lateral generally keel-like portion 360 extending generally inferiorly from plate 120 and radiating generally laterally from collar 300, such that the medial extension of portion 340 is angularly disposed from the lateral extension of portion 360 by an angle 380 of about 180 degrees (see FIG. 3). In alternative embodiments, the keel-like portions of base 280 may suitably radiate or fan out from collar 300 at any other suitable angle or angles. Moreover, in alternative embodiments base 280 may include any suitable number of keel-like portions (including none at all in some embodiments) and/or any suitable number of other suitably positioned suitable protuberance(s) (e.g., spikes, vanes, etc.) for suitably anchoring base 280 into the proximal tibia.

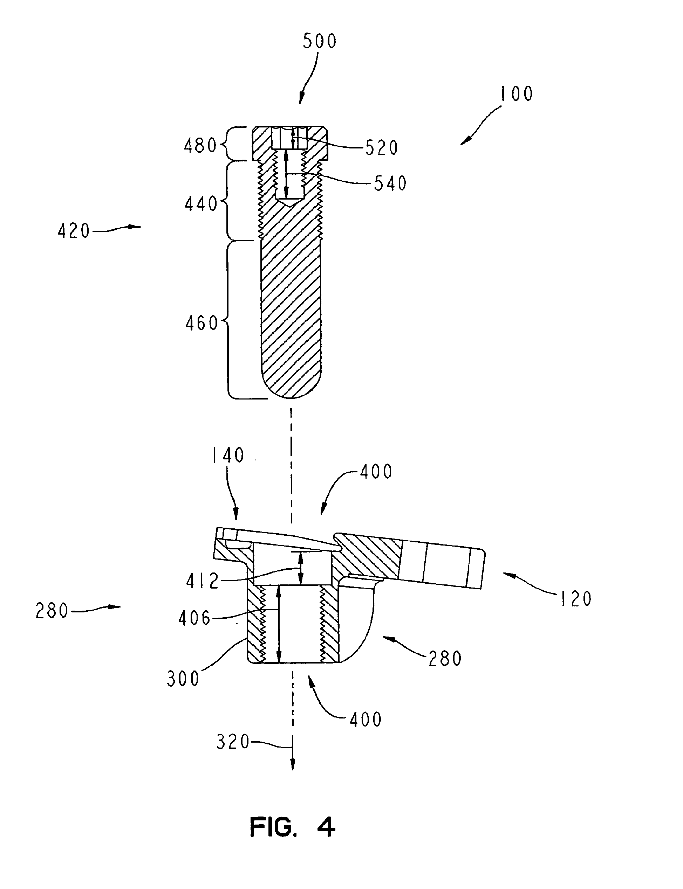

Plate 120 and collar 300 define a passageway or through-channel 400 (see FIG. 3 and FIG. 4) axially extending all the way through surface 160 and collar 300 along axis 320. Through-channel 400 includes a generally cylindrically-shaped screw-threaded portion 406 and a generally cylindrically-shaped non-threaded portion 412 (see FIG. 4).

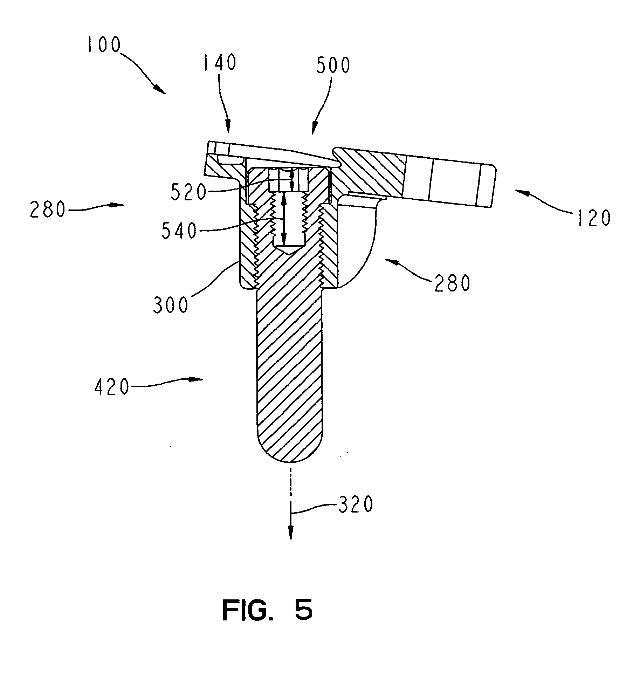

Implant 100 further includes an exemplary extension member or post 420 inserted through and extending from through-channel 400 along axis 320 (see FIG. 4 and FIG. 5). Among other things, post 420 is configured to couple to base 280 within through-channel 400 and to anchor into the proximal tibia such that post 420 (and, thus, implant 100) is linearly fixed relative to the proximal tibia along axis 320. Post 420 includes a generally cylindrically-shaped screw-threaded shaft portion 440 screw-coupled to base 280 within portion 406 of through-channel 400 (see FIG. 4). Post 420 further includes a generally cylindrically-shaped non-threaded shaft portion 460 extending generally inferiorly out of through-channel 400 along axis 320, and a head portion 480 generally opposite portion 460 (see FIG. 4). Portion 480 is retained in portion 412 of through-channel 400 (see FIG. 4). In alternative embodiments, portion 440 of post 420, portion 480 of post 420, portion 406 of through-channel 400, and/or portion 412 of through-channel 400 may be alternatively configured to provide a taper coupling or any other suitable coupling between post 420 and base 280.

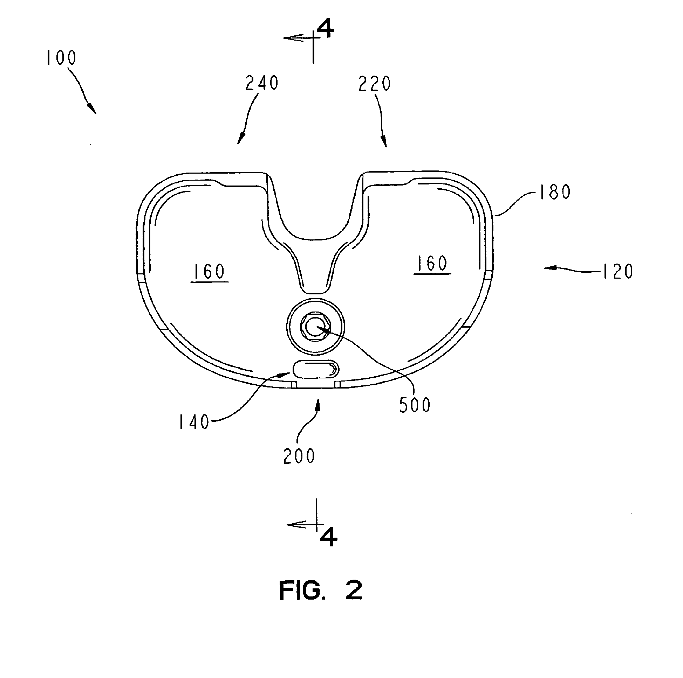

Further, post 420 defines a socket 500 (see also FIG. 2, FIG. 4, and FIG. 5) having a non-circular generally cylindrically-shaped portion 520 opening from head 480 and extending into post 420, and further having a generally cylindrically-shaped screw-threaded portion 540 extending from portion 520 even further into post 420 (see FIG. 4 and FIG. 5). Among other things, portion 520 of socket 500 is configured to facilitate installation of post 420 in through-channel 400 of base 280. Portion 540 of socket 500 facilitates the alignment and/or retention of insert 80. In alternative embodiments, portion 520 may be hexagonally-shaped, torqx-shaped, or otherwise suitably alternatively shaped, and/or portion 540 may be configured for a taper or any other suitable coupling.

FIG. 2 shows a superior plan view of exemplary tibial implant 100. Among other things, slot 140, surface 160, wall 180, notch 200, and socket 500 are discernable in FIG. 2.

FIG. 3 shows an inferior plan view of exemplary tibial plate 120 and exemplary base 280. Among other things, collar 300, medial generally keel-like portion 340, lateral generally keel-like portion 360, angle 380, and through-channel 400 are discernable in FIG. 3.

FIG. 4 shows an exploded cross-sectional view of exemplary tibial implant 100 (taken along line 4-4 of FIG. 2). Among other things, portion 406 of through-channel 400, portion 412 of through-channel 400, portion 440 of post 420, portion 460 of post 420, portion 480 of post 420, portion 520 of socket 500, and portion 540 of socket 500 are discernable in FIG. 4. FIG. 5 shows an assembled cross-sectional view of exemplary tibial implant 100 (taken along line 4-4 of FIG. 2).

To use exemplary apparatus 60, the knee joint is opened and the proximal tibia is prepared to receive implant 100 via suitable minimally invasive surgical techniques or any other suitable procedures. Post 420 is removed from through-channel 400 (and, thus, separated from base 280). The proximal tibia is spaced apart from the distal femur as necessary to provide clearance for inserting plate 120 and base 280 into the joint space and aligning them superior to the proximal tibia. It should be appreciated that the necessary clearance space may be less than that which would be required to insert and align plate 120 and base 280 with post 420 installed.

Next, bone cement is suitably applied to the superior surface of the prepared proximal tibia. Plate 120 and base 280 are suitably aligned superior to the proximal tibia (with, among other things, through-channel 400 suitably generally coaxially aligned with the longitude of the tibial intramedullary canal along axis 320), and base 280 is hammered or otherwise suitably forced generally distally into the proximal tibia. Among other things, this anchors base 280 into the proximal tibia such that base 280 (and, thus, plate 120) is rotationally fixed relative to the proximal tibia about axis 320.

Next, post 420 is inserted through through-channel 400 along axis 320, hammered or otherwise suitably forced generally distally and generally longitudinally into the intramedullary canal until screw-threaded portion 440 of post 420 reaches screw-threaded portion 406 of through-channel 400. Further, a suitable hexagonal drill bit, suitable hexagonal screwdriver head, suitable Allen wrench, or any other suitable tool is inserted into portion 520 of socket 500 and torqued to screw portion 440 of post 420 into portion 406 of through-channel 400 (thus forcing post 420 even further generally distally and generally longitudinally into the intramedullary canal) until head portion 480 of post 420 suitably seats within portion 412 of through-channel 400. Among other things, this anchors post 420 into the proximal tibia such that post 420 (and, thus, implant 100) is linearly fixed relative to the proximal tibia along axis 320. After anchoring post 420 into the proximal tibia, the torquing tool is withdrawn from socket 500.

Insert 80 is aligned and/or retained on surface 160 of plate 120 via slot 140 of plate 120, wall 180 of plate 120, notch 200 of plate 120, and/or portion 540 of socket 500.

The foregoing description of the invention is illustrative only, and is not intended to limit the scope of the invention to the precise terms set forth. Further, although the invention has been described in detail with reference to certain illustrative embodiments, variations and modifications exist within the scope of the invention as defined in the following claims.

## Claims

1. A tibial implant apparatus (100), comprising:
a tibial plate (120);
a tibio-femoral insert (80) which couples to the tibial plate;
a base (280) extending from the tibial plate (120); and
an elongated member (420) including a first portion (440) and a second portion (460), said elongated member (420) being removably attached to said base (120);
wherein the tibial plate (120) and the base (280) cooperatively define a through-channel (400), the elongated member (420) is inserted superiorly into the through-channel (400), such that the first portion (440) is retained within the through-channel and the second portion (460) of the elongated member protrudes out of said through-channel and extends inferiorly away from the base (280); **characterised in that**
the elongated member (420) defines a socket (500) having a portion (540) serving to align and/or retain the tibio-femoral insert (80).

2. The apparatus of claim 1, wherein the first portion (440) of the elongated member (420) is removably retained in the through-channel.

3. The apparatus of any of claims 1 or 2, wherein the tibial plate (120) includes a medial portion (220) and a lateral portion (240), and the through-channel (400) is configured to receive the elongated member (420) from a point generally superior to the tibial plate (120).

4. The apparatus of any of claims 1 to 3, wherein the base (280) includes a first generally keel-like portion (340; 360).

5. The apparatus of claim 4, wherein the first generally keel-like portion extends generally radially outwardly relative to the through-channel (400).

6. The apparatus of claim 4 or 5, wherein the base includes a second generally keel-like portion (360; 340) extending generally radially outwardly relative to the through-channel at an angle of about 180 degrees from the radially outwardly extension of the first generally keel-like portion (340; 360).

7. The apparatus of any of claims 1 to 6, wherein the first portion (440) of the elongated member (420) is retained in the through-channel (400) at least in part by at least one of screwing the first portion (440) of the elongated member into the base (280) and tapering the first portion of the elongated member into the base.

8. The apparatus of any of claims 1 to 7, wherein the socket (500) includes at least one of a generally hexagonally-shaped portion (520) and a generally torqx-shaped portion.

9. The apparatus of claim 8, wherein the socket (500) includes at least one of a screw-threaded portion (540) and a tapered portion.

10. The apparatus of any of claims 1 to 9, wherein the base (280) includes a plurality of protuberances (340; 360) positioned generally radially outwardly away from the through-channel (400).

## Patentansprüche

1. Tibiaimplantatvorrichtung (100), umfassend:
eine Tibiaplatte (120);
einen Tibia-Femur-Einsatz (80), der mit der Tibiaplatte gekoppelt ist;
eine Basis (280), die sich von der Tibiaplatte (120) erstreckt; und
ein lang gestrecktes Element (420), das einen ersten Abschnitt (440) und einen zweiten Abschnitt (460) umfasst, wobei das lang gestreckte Element (420) entfernbar an der Basis (120) befestigt ist;
wobei die Tibiaplatte (120) und die Basis (280) zusammenwirkend einen Durchgangskanal (400) definieren, wobei das lang gestreckte Element (420) von oben in den Durchgangskanal (400) eingesetzt ist, so dass der erste Abschnitt (440) in dem Durchgangskanal gehalten ist und der zweite Abschnitt (460) des lang gestreckten Elements aus dem Durchgangskanal herausragt und sich nach unten von der Basis (280) weg erstreckt; **dadurch gekennzeichnet, dass**
das lang gestreckte Element (420) einen Sockel (500) mit einem Abschnitt (540) definiert, der dazu dient, den Tibia-Femur-Einsatz (80) auszurichten und/oder zu halten.

2. Vorrichtung nach Anspruch 1, wobei der erste Abschnitt (440) des lang gestreckten Elements (420) entfernbar in dem Durchgangskanal gehalten ist.

3. Vorrichtung nach Anspruch 1 oder 2, wobei die Tibiaplatte (120) einen medialen Abschnitt (220) und einen lateralen Abschnitt (240) umfasst und der Durchgangskanal (400) ausgestaltet ist, um das lang gestreckte Element (420) von einer Stelle im Wesentlichen oberhalb der Tibiaplatte (120) aufzunehmen.

4. Vorrichtung nach einem der Ansprüche 1 bis 3, wobei die Basis (280) einen ersten im Wesentlichen kielähnlichen Abschnitt (340; 360) umfasst.

5. Vorrichtung nach Anspruch 4, wobei sich der erste im Wesentlichen kielähnliche Abschnitt relativ zu dem Durchgangskanal (400) im Wesentlichen radial nach außen erstreckt.

6. Vorrichtung nach Anspruch 4 oder 5, wobei die Basis einen zweiten im Wesentlichen kielähnlichen Abschnitt (360; 340) umfasst, der sich relativ zu dem Durchgangskanal unter einem Winkel von etwa 180 Grad zu der radial nach außen gerichteten Erstreckung des ersten im Wesentlichen kielähnlichen Abschnitts (340; 360) im Wesentlichen radial nach außen erstreckt.

7. Vorrichtung nach einem der Ansprüche 1 bis 6, wobei der erste Abschnitt (440) des lang gestreckten Elements (420) in dem Durchgangskanal (400) zumindest teilweise durch Einschrauben des ersten Abschnitts (440) des lang gestreckten Elements in die Basis (280) und/oder sich in die Basis hinein Verjüngen des ersten Abschnitts des lang gestreckten Elements gehalten ist.

8. Vorrichtung nach einem der Ansprüche 1 bis 7, wobei der Sockel (500) einen im Wesentlichen hexagonalförmigen Abschnitt (520) und/oder einen im Wesentlichen torxförmigen Abschnitt umfasst.

9. Vorrichtung nach Anspruch 8, wobei der Sockel (500) einen Schraubgewindeabschnitt (540) und/oder einen sich verjüngenden Abschnitt umfasst.

10. Vorrichtung nach einem der Ansprüche 1 bis 9, wobei die Basis (280) mehrere Vorsprünge (340; 360) umfasst, die von dem Durchgangskanal (400) im Wesentlichen radial nach außen weggerichtet positioniert sind.

## Revendications

1. Appareillage formant implant tibial (100), comprenant :
un plateau tibial (120) ;
un insert tibial-fémoral (80) qui est couplé au plateau tibial ;
une base (280) s'étendant depuis le plateau tibial (120) ; et
un élément allongé (420) qui inclut une première portion (440) et une seconde portion (460), ledit élément allongé (420) étant attaché de façon amovible sur ladite base (120) ;
dans lequel le plateau tibial (120) et la base (280) coopèrent pour définir un canal traversant (400), l'élément allongé (420) étant inséré depuis la partie supérieure dans le canal traversant (400), de telle sorte que la première portion (440) est retenue à l'intérieur du canal traversant, et la seconde portion (460) de l'élément allongé se projette hors dudit canal traversant et s'étend en direction inférieure en éloignement de la base (280) ;
**caractérisé en ce que**
l'élément allongé (420) définit un socle (500) comprenant une portion (540) qui sert à aligner et/ou à retenir l'insert tibial-fémoral (80).

2. Appareil selon la revendication 1, dans lequel la première portion (440) de l'élément allongé (420) est retenue de façon amovible dans le canal traversant.

3. Appareil selon l'une quelconque des revendications 1 et 2, dans lequel le plateau tibial (120) inclut une portion médiane (220) et une portion latérale (240), et le canal traversant (400) est configuré pour recevoir l'élément allongé (420) depuis un point généralement supérieur par rapport au plateau tibial (120).

4. Appareil selon l'une quelconque des revendications 1 à 3, dans lequel la base (280) inclut une première portion (340 ; 360) généralement semblable à une quille.

5. Appareil selon la revendication 4, dans lequel la première portion généralement semblable à une quille s'étend généralement radialement vers l'extérieur par rapport au canal traversant (400).

6. Appareil selon la revendication 4 ou 5, dans lequel la base inclut une seconde portion (360 ; 340) généralement semblable à une quille, s'étendant généralement radialement vers l'extérieur par rapport au canal traversant sous un angle d'environ 180° par rapport à l'extension radiale vers l'extérieur de la première portion (340 ; 360) généralement semblable à une quille.

7. Appareil selon l'une quelconque des revendications 1 à 6, dans lequel la première portion (440) de l'élément allongé (420) est retenue dans le canal traversant (400) au moins en partie par une méthode au moins parmi le vissage de la première portion (440) de l'élément allongé dans la base (280), et l'enfoncement de la première portion de l'élément allongé dans la base.

8. Appareil selon l'une quelconque des revendications 1 à 7, dans lequel le socle (500) inclut au moins une portion parmi une portion de forme générale hexagonale (520) et une portion de forme générale dite "Torx".

9. Appareil selon la revendication 8, dans lequel le socle (500) inclut au moins une portion parmi une portion filetée (540) et une portion en pointe.

10. Appareil selon l'une quelconque des revendications 1 à 9, dans lequel la base (280) inclut une pluralité de protubérances (340 ; 360) positionnées généralement radialement à l'extérieur en éloignement du canal traversant (400).
